# EUROPEAN PATENT SPECIFICATION

(11) **EP 4 087 834 B1**
(45) Date of publication and mention of the grant of the patent: **22.11.2023**
(21) Application number: 20841732.9
(22) Date of filing: 29.12.2020
(51) Int. Cl.: C07C 51/235, C07C 51/44, C07C 53/122, C07C 51/487

(54) **PROCESS FOR THE PREPARATION OF C3-5 SATURATED ALIPHATIC CARBOXYLIC ACIDS**
VERFAHREN ZUR HERSTELLUNG VON GESÄTTIGTEN ALIPHATISCHEN C3-5-CARBONSÄUREN
PROCÉDÉ DE PRÉPARATION D'ACIDES CARBOXYLIQUES ALIPHATIQUES SATURÉS EN C3 À C5

(30) Priority: 09.01.2020 EP 20150845
(43) Date of publication of application: 16.11.2022
(73) Proprietor: BASF SE, 67056 Ludwigshafen am Rhein (DE)
(72) Inventor: TELES, Joaquim, Henrique, 67059 Ludwigshafen am Rhein (DE); HAMANN, Jessica, Nadine, 67059 Ludwigshafen am Rhein (DE); LEFEBVRE, Jonathan, Thomas, 67059 Ludwigshafen am Rhein (DE); WEISSKER, Wolf-Steffen, 67059 Ludwigshafen am Rhein (DE); LIANG, Shelue, 67059 Ludwigshafen am Rhein (DE)
(74) Representative: BASF IP Association
(86) International application number: PCT/EP2020/087952
(87) International publication number: WO 2021/140044

(56) References cited:
- CN-A- 110 526 814
- FR-A1- 2 609 025
- JP-A- 2001 011 009
- US-A- 4 350 829
- US-A- 4 510 331

## Description

The present invention relates to a process for the preparation of a saturated aliphatic carboxylic acid with 3 to 5 carbon atoms by oxidation of the corresponding aldehyde with oxygen, in which the saturated aliphatic carboxylic acid is obtained in a high purity with regard to active oxygen containing compounds.

Saturated aliphatic carboxylic acids are important intermediates globally with a wide range of applications. They can be used as such, but are typically further processed to metal salts, esters, amides, anhydrides, acid chlorides, and other synthesis components. Overall, they are an important intermediate for the production of variety of compounds such as metal salts and metal soaps, flavors, fragrances, drug synthesis, cosmetic ingredients, plasticizers, paints, coating additives, coolants, lubricants or catalysts for polymer processing. Propionic acid as one very important representative of saturated aliphatic carboxylic acids is mainly used as a preservative for animal feed and food for human consumption.

A widely used and important method for the production of saturated aliphatic carboxylic acids with 3 to 5 carbons atoms is the oxidation of the corresponding aldehydes with molecular oxygen in the liquid phase in the presence or absence of a catalyst. The production of propionic acid by oxidation of propionaldehyde, for example, is described as an important route in U.-R. Samel et al., "Propionic Acid and Derivatives" in Ullmann's Encyclopedia of Industrial Chemistry, 2017, Wiley-VCH Verlag GmbH & Co. KGaA, DOI: 10.1002/14356007.a22_223.pub4, chapter 4.2 "Oxidation of Propanal".

CN 108707071 describes a specific process for the production of propionic acid by oxidation of propionaldehyde, in which propionaldehyde is first contacted in a high-pressure reactor with a Co containing oxidation catalyst at 30 to 50°C and then heated to 85 to 110°C while pressurizing the reactor with oxygen. The reactor was then kept under these conditions for a reaction time of 2 to 3 hours and the reaction mixture subsequently distilled to obtain propionic acid.

CN110526814 discloses a process for the preparation of butyric acid by oxidation of butyraldehyde with air, which comprises (a) converting butyraldehyde with air in an oxidation tower at a temperature of 30 to 40°C and a molar ratio of butyraldehyde to air of 0.8 to 0.9 to obtain a mixture containing butyraldehyde and butyric acid, (b) condensing the mixture withdrawn at the top of the oxidation tower in a condenser, (c) conveying the liquid to a rectification tower and heating it therein, (d) condensing the butyraldehyde withdrawn from the top of the rectification tower and returning it to the oxidation tower, and (e) forwarding the n-butyric acid to a storage tank.

It is known from the state of the art that the oxidation of aldehydes with oxygen initially forms peroxy acids which then oxidize further aldehyde to produce two moles of carboxylic acids per mole of intermediate peroxy acids. Such a mechanism is, for example, described in J.H. Teles et al., "Oxidation" in Ullmann's Encyclopedia of Industrial Chemistry, 2015, Wiley-VCH Verlag GmbH & Co. KGaA, DOI: 10.1002/14356007.a18_261.pub2, chapter 2 "Organic Chemical Production by Oxidation". It is therefore apparent that peroxides occur as intermediates during the oxidation of aldehydes with oxygen as well as remaining byproducts in carboxylic acids which have been produced by the oxidation of aldehydes. Peroxides are very reactive molecules and, in higher concentrations, also tend to explode. Hence, it is expedient to also have an eye on the formation of peroxides when oxidizing aldehydes with oxygen.

DE 1,071,685 deals with the preparation of short-chained saturated aliphatic carboxylic acids with 2 to 4 carbon atoms by oxidation of the respective aldehydes. It depicts that without a catalyst, the oxidation reaction might break down at low temperatures around 30°C and consequently form an explosive mixture comprising the non-reacted aldehyde and excess oxygen. In addition to that, also the peroxy acids which are initially formed by the addition of O₂ to aldehydes would accumulate, since their reactivity at such low temperatures is quite low. This may lead to a dangerous enrichment of explosive peroxides. A higher reaction temperature above 70°C would accelerate the oxidation process, as well as increase the reactivity of the peroxides, but also leads to an increase of unwanted byproducts due to increasing unselective thermal decomposition of the peroxides. At the end, DE 1,071,685 teaches to use an alkali hydroxide catalyst to accelerate the reaction at lower temperatures and to avoid the formation of a gaseous phase. Thus, the risk of forming dangerous amounts of peroxides can be avoided.

US 3,579,575 teaches another way to reduce the concentration of peroxides. It is first described that the oxidation of branched short-chain aldehydes or olefinically unsaturated short-chain aldehydes with 4 to 5 carbon atoms lead in the presence of an oxidation catalyst to a comparatively low yield and an undesired yellow color of the carboxylic acids so produced. Furthermore, it also describes that such inconveniences can be avoided by carrying out the oxidation in the absence of a catalyst. However, in such a case, the peroxide concentration would become very high which may cause serious accidents such as explosions. In order to avoid this risk, the US patent proposes to carry out the oxidation in the presence of water in an amount sufficient to form a separate aqueous liquid phase. It is assumed that the polar peroxide radicals are mainly at the interface between the organic and the aqueous phase directed to the aqueous phase, whereby the hydrocarbon chain remains at the side of the organic phase. Accordingly, the peroxides would decompose into the aqueous phase and prevent the native oxygen resulting from such decomposition to uncontrollably react with the hydrocarbon chain.

Although the concentration of peroxides might be significantly reduced by such a measure, it is disadvantageous to dilute the reaction mixture with water. First, the additional component would enlarge the reaction volume which would require a larger reactor. Second, the aqueous phase has to be separated off, which would require a separator or settling tank as a further apparatus. The separated aqueous phase would, of course, also contain some carboxylic acid, which would be lost in the total yield and which has to be disposed as chemical waste, causing further efforts. Third, the corrosivity of carboxylic acids in the presence of water is much higher than in the absence of water. If water is present, complex corrosion resistant construction materials are required. And last but not least, even if the aqueous phase is separated off, the organic phase would also contain some water dissolved therein. This would make the purification of the carboxylic acid by distillation more complex. It also bears the risk that the purified carboxylic acid might still contain higher amounts of water than it would contain without adding water in the oxidation. Since water is usually a specified byproduct in the pure acid, the addition of water requires additional effort to get a specification abiding product.

In CN 108047027, it was found that the concentration of the peroxides formed in the oxidation of 3,5,5-trimethylhexanal to 3,5,5-trimethylhexanoic acid (isononanoic acid) is rather low in the reaction stage, so that the explosion risk is no issue there, but that the rather low concentration is still high enough to cause problems in the rectification column since the peroxides may accumulate therein due to the difference in the boiling points of the peroxy acid and other components. Furthermore, the CN application describes that in the prior art, homogeneous catalysts in a relatively low concentration are used to decompose the peroxides. However, such homogeneous catalysts are very difficult to separate off and also cause the risks of slagging, clogging and explosions in the distillation column. To avoid such problems, the CN application teaches to heterogeneously catalyze the decomposition of the peroxides over a metal organic framework catalyst before entering the rectification column. It is taught to be crucial to only decompose the peroxides at a low temperature of 20 to 70°C since otherwise side reactions such as decarboxylation would appear and lower the yield and purity. Moreover, it is emphasized that the decomposition is very fast and therefore can be carried out at a high space velocity of 5 to 40 h⁻¹, which relates to a short residence time of 1.5 to 12 minutes.

Irrespective of the fact that the CN application is specifically directed to the production of isononanoic acid, it is generally disadvantageous to use a metal organic framework catalyst as peroxide decomposition catalyst. First of all, these metal organic framework catalysts are quite complex to produce. Second, the organic molecules that build the framework are susceptible to oxidation, especially in the presence of a peroxy acid and of the reactive radicals generated in their decomposition. In the presence of carboxylic acids, metal organic frameworks are known to lose active and/or framework metals as a result of bleeding. These leached metals would then cause the same problems in the rectification column as metals used as homogeneous catalyst. Furthermore, all these factors contribute to a short lifetime of the metal organic framework catalysts and thus to an additional increase of the complexity of the process by disposing the spent catalyst and providing fresh one.

Regarding the production of propionic acid by oxidation of propionaldehyde, it was recognized according to the present invention that high conversions lead to an increase of the concentration of perpropionic acid. Since perpropionic acid has only a slightly lower boiling point than propionic acid and is quite stable, it becomes difficult to obtain pure propionic acid with a very low concentration of perpropionic acid. Perpropionic acid is a strong oxidizing agent which might cause serious problems in the downstream processing of propionic acid. For example, it is known that propionic acid with an elevated content of perpropionic acid of 100 wt.-ppm or more leads to an undesired coloration when such propionic acid is processed to ester products. The above-mentioned behavior does in principle also apply for the production of the respective C₄ and C₅ carboxylic acids.

Therefore, it is desirable to provide C₃₋₅ carboxylic acids without or at least with a very low content of peroxy acid and other peroxides.

It was an object of the present invention to find a process for the preparation of saturated aliphatic carboxylic acids with 3 to 5 carbon atoms by oxidation of the corresponding aldehyde with oxygen, which is able to produce the respective saturated aliphatic carboxylic acid in high yield and high purity, and particularly without or at least with a very low content of peroxy acid and other peroxides. The process shall also be easy to operate and function stably over long operating times producing the saturated aliphatic carboxylic acids in a constant high quality.

A process for the preparation of a saturated aliphatic non-alpha-branched carboxylic acid with 3 to 5 carbon atoms by oxidation of the corresponding aldehyde with oxygen has been found, which comprises
(a) converting the corresponding aldehyde with oxygen at a temperature of 40 to 150°C and an oxygen partial pressure of 0.001 to 1 MPa to obtain a mixture containing the saturated aliphatic carboxylic acid and ≤ 2 mol-% of the corresponding aldehyde with respect to the saturated aliphatic carboxylic acid,
(b) thermally treating the mixture obtained in step (a) in the liquid phase at a temperature of 80 to 250°C and a pressure of 0.1 to 2 MPa abs for 0.25 to 100 hours, and
(c) distilling the mixture obtained in step (b) in single distillation column to obtain a distillate containing ≥ 90 wt.-% of the saturated aliphatic carboxylic acid and having an active oxygen content of 0 to 25 wt.-ppm based on the distillate.

The saturated aliphatic carboxylic acids for which this production process is very suitable contain 3 to 5 carbon atoms. The carboxylic acids may also contain heteroatoms such as halogen instead of hydrogen, but non-substituted carboxylic acids are preferred. As examples of C₃₋₅ saturated non-alpha-branched aliphatic carboxylic acids, propionic acid, n-butyric acid, n-pentanoic acid (valeric acid) and 3-methylbutyric acid are mentioned. Particularly preferably propionic acid is prepared.

In the first step of the process according to the invention, which is named step (a), the corresponding aldehyde is oxidized with oxygen. According to the C₃₋₅ saturated aliphatic carboxylic acid to be produced, the corresponding aldehyde is propionaldehyde, n-butyraldehyde, 2-methylpropionaldehyde (isobutyraldehyde), n-pentanaldehyde (valeraldehyde), 3-methylbutyraldehyde, 2-methylbutyraldehyde or 2,2-dimethylpropionaldehyde (pivalic aldehyde). According to the preferred production of non-alpha-branched carboxylic acids, propionaldehyde, n-butyraldehyde or n-pentanaldehyde are the corresponding aldehyde.

The aldehyde can be used in diluted or pure form. If the aldehyde is used in a diluted form, the diluent shall preferably be a compound which is inert against an oxidation with oxygen, stable against the produced carboxylic acid and easily separated from the carboxylic acid by distillation. Since diluents raise the reaction volume and therefore lower the space time yield, and additionally may cause a contamination of the wanted carboxylic acid, it is preferred not to intentionally dilute the aldehydes. Preferably, the aldehyde is applied as a highly concentrated compound with an aldehyde content of preferably 80 to 100 wt.-%, more preferably of 80 to 100 wt.-%, particularly preferably of 95 to 100 wt.-% and very particularly preferably of 99 to 100 wt.-%.

Besides the preparation of only one specific saturated aliphatic carboxylic acid, the inventive process can also be applied for the preparation of mixtures of saturated aliphatic carboxylic acids. Mixtures of technical interest are for example a mixture of n-pentanoic acid and 2-methylbutyric acid, for which n-pentanaldehyde and 2-methylbutyraldehyde are used, or a mixture of n-butyric acid and isobutyric acid, for which n-butyraldehyde and isobutyraldehyde are used.

Particularly preferred is the preparation of propionic acid by oxidation of propionaldehyde.

The oxidation of the aldehyde is performed with oxygen. It can be used in pure form or diluted with other gases, for example in the form of air, an O₂/N₂ mixture or in form of mixtures with other inert gases.

The oxidation reaction may be carried out in the presence or absence of an oxidation catalyst and/or in the presence or absence of a selectivity improving additive. If oxidation catalysts are used, they will be homogeneous catalysts. As examples of homogeneous oxidation catalysts, salts of transition metals of groups 6 to 11 of the Periodic Table of the Elements are mentioned, preferably of the first row of these groups and most preferably Mn, Fe or Co salts. If selectivity improving additives are used, they will also be homogeneous. As examples of selectivity improving additives, salts of alkali metals, of alkaline earth metals and of transition metals of group 12 of the Periodic Table of the Elements are mentioned, preferably salts of Na, K, Mg, Ca, Zn or Cd and most preferably salts of K or Na. The salts can be chosen from any salts soluble in the reaction mixture, but carboxylates, hydroxides, carbonates and hydrogen carbonates are preferred. The concentration of the homogeneous oxidation catalyst metals may vary in a broad range, but a metal content of 0.0001 to 0.1 wt.-% based on the reaction mixture is a typical content. The concentration of the homogeneous selectivity improving metals may also vary in a broad range, but a metal content of 0.1 to 5 wt.-% based on the reaction mixture is a typical content. It is also possible to simultaneously use homogeneous catalyst metals and homogeneous selectivity improving metals.

Depending on the nature of the aldehyde, the presence of an oxidation catalyst and particularly the presence of selectivity improving metals will influence the range and type of the by-products. For example, alpha-branched aldehydes like isobutyraldehyde or 2-methylbutyraldehyde tend to produce more formates as by-product if no selectivity improving metal is present. However, in the presence of a selectivity improving metal, particularly in the presence of a sodium or potassium salt and most particularly in the presence of a potassium salt, alpha-branched aldehydes produce much less unwanted formates. Thus, it is preferred for alpha-branched aldehydes to conduct the oxidation in the presence of a selectivity improving metal, preferably in the presence of a sodium or potassium salt and particularly preferably in the presence of a potassium salt. On the other hand, linear aldehydes like propionaldehyde, n-butyraldehyde and n-pentanaldehyde, already form in the absence of a selectivity improving metal only a small amount of formates, so that the addition of a selectivity improving metal is not or only less relevant for the selectivity. Thus, it is preferred for linear aldehydes to conduct the oxidation in the absence of a selectivity improving metal.

As for homogeneous catalytic metals, they increase the reaction rate, but they have a detrimental effect on selectivity. It is thus preferred to perform the oxidation in the absence of added homogeneous catalyst metals.

Irrespective of whether an oxidation catalyst is present or not, the oxidation reaction is performed at a temperature of 40 to 150°C and an oxygen partial pressure of 0.001 to 1 MPa. It is preferably carried out at a temperature of ≥ 50°C, more preferably at ≥ 60°C and particularly preferably at ≥ 70°C, and preferably at ≤ 120°C, more preferably at ≤ 100°C and particularly preferably at ≤ 80°C. With regard to the oxygen partial pressure, it is preferably carried out at an oxygen partial pressure of ≥ 0.005 MPa, more preferably of ≥ 0.01 MPa and particularly preferably of ≥ 0.02 MPa, and preferably of ≤ 0.8 MPa and more preferably of ≤ 0.5 MPa. The oxygen partial pressure can easily be determined by measuring the total pressure and multiplying by the concentration of O₂ in vol.-% determined by any suitable method known in the state of art.

Whereas the oxygen partial pressure may vary in a broad range based on the oxygen content of the oxygen source, the total pressure at step (a) is usually in the range of 0.05 to 5 MPa abs. The oxidation reaction is preferably carried out at a total pressure of ≥ 0.08 MPa abs, more preferably at a total pressure of ≥ 0.09 MPa abs and particularly preferably at a total pressure of ≥ 0.1 MPa abs. It is preferably carried out at a total pressure of ≤ 4 MPa abs, more preferably at a total pressure of ≤ 3 MPa abs and particularly preferably at a total pressure of ≤ 2.5 MPa abs.

At these conditions, the aldehydes are almost completely in the liquid phase and the oxidation reaction also takes place in the liquid phase.

The oxidation of the aldehyde in step (a) is typically performed in a reaction device, either batch-wise, semi-continuously or continuously. At a continuous operation, aldehyde and oxygen are continuously fed to the reaction device and an adequate stream of the reaction mixture is continuously removed. The process conditions of the continuous operation, including the residence time, are selected in such a way that the desired conversion is achieved. At a batch operation, the reaction device is filled with aldehyde and oxygen added and, if required, replenished.

After having achieved the desired conversion, the mixture is removed from the reaction device. A semi-continuous operation is characterized by adding aldehyde and oxygen together or intermittently to the reaction device over a specific period of time while the oxidation reaction already takes place. After a while, e.g. if the reaction device is more or less filled, the addition is stopped, and after having achieved the desired conversion, the mixture is removed from the reaction device.

The preferred operations for step (a) are batch operation and continuous operation, whereby the continuous operation is particularly preferred.

The oxidation reaction is typically performed in a reaction device. The reaction device may embrace one or more reaction apparatuses. In principle, suitable reaction apparatuses to be used in the reaction device according to the invention include those which are suitable for carrying out exothermic, gas-liquid reactions and which can be operated discontinuously, semi-continuously or continuously. For a discontinuous process, stirred autoclaves or autoclaves with a jet loop type mixing are for example suitable. For a semi-continuous process, stirred vessels, trickle-bed reactors, and bubble column reactors are mentioned as possible examples. For a continuous process, stirred vessels, trickle-bed reactors, bubble column reactors, jet loop reactors and cascades of the above-mentioned reactors are mentioned as suitable examples. Preferred examples of suitable reactors are described in more detail in WO 2009/024,446 and WO 2009/024,549. If reactor cascades are used, as for example in a continuous process, 2 to 5, preferably 2 to 4, and particularly preferably 2 to 3 reactors connected in sequence.

It is preferred to use reaction apparatuses which enable an intensive gas-liquid mixing and a good distribution of the oxygen within the liquid reaction mixture.

Due to formation of a considerable amount of reaction heat by the oxidation, it is necessary to remove the heat from the reaction zone. Depending on the concentration of the aldehyde and the oxygen fed into the reactor, it may suffice in a continuously performed process to remove the heat only with the reaction mixture and to control the temperature in the reactor by adding fresh aldehyde with a low temperature. However, for aldehyde in a higher concentration and an oxygen content in the order of air or above, it is normally required to cool the reaction liquid in the reactor. Such cooling may for example be performed by an externally cooled exterior wall of the reactor or by cooling tubes inside the reactor, through which a coolant is flowed.

According to the overall chemical equation 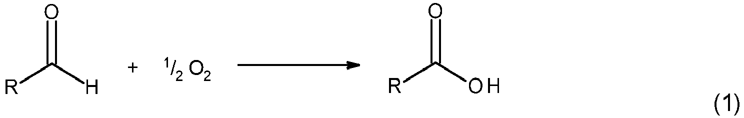 in which R denotes the C₂₋₄ radical of the C₃₋₅ aldehyde and the C₃₋₅ carboxylic acid, 0.5 mol of oxygen O₂ is stoichiometrically required to oxidize the aldehyde to the carboxylic acid. Although it would be possible to perform the oxidation with a deficit of oxygen with the result of a partial conversion and the existence of residual aldehyde in the reaction mixture, it is preferred to apply oxygen in stoichiometric or superstoichiometric amount. In order to ensure an adequate conversion at the one hand and to limit the gas load at the other hand, the oxidation reaction is preferably performed at a molar ratio of oxygen to aldehyde of 0.5 to 1. It is more preferably performed at a molar ratio of oxygen to aldehyde of ≥ 0.51 and particularly preferably of ≥ 0.52, and more preferably at ≤ 0.7, particularly preferably of ≤ 0.6 and very particularly preferably of ≤ 0.58.

It has been shown that for a continuous oxidation process, the use of a reactor cascade is particularly advantageous since it enables the stepwise addition of oxygen. The great advantages of such a stepwise addition of oxygen are a better control of the reaction heat and particularly a lower amount of by-products due to a lower oxygen concentration in the respective reactor. Therefore, it is particularly preferred to use such a reactor cascade of 2 to 3 reactors, whereby preferably the total amount of the aldehyde and about 70 to 95% of the total oxygen are fed to the first reactor and the remaining 5 to 30% of the oxygen either in total to the second reactor or further divided into two parts to the second and third reactor, whereby the fraction for the third reactor would then be the smaller fraction.

Even though the oxygen is applied in stoichiometric or superstoichiometric amount, it would take a very long time to achieve an aldehyde conversion of nearly 100%. This would unnecessarily block the reaction equipment or make it extremely large. It is therefore advantageous to convert in step (a) the aldehyde until a residual aldehyde amount of ≤ 2 mol-% with respect to the saturated aliphatic carboxylic acid is achieved. Depending on the nature of the aldehyde, the concentration of the oxygen containing gas provided to the reaction device, and the process conditions, a residual aldehyde amount of ≤ 2 mol-% with respect to the saturated aliphatic carboxylic acid is generally achieved after a reaction time of 0.1 to 5 hours.

The mixture obtained in step (a) preferably contains ≤ 1.5 mol-% and more preferably ≤ 1 mol-%, and preferably ≥ 0.1 mol-%, more preferably ≥ 0.2 mol-% and particularly preferably ≥ 0.5 mol-% of the corresponding aldehyde with respect to the saturated aliphatic carboxylic acid.

As the reaction time concerns, a time of ≥ 0.2 hours is preferred, ≥ 0.3 hours are more preferred and ≥ 0.5 hours are preferably preferred. Furthermore, a time of ≤ 4 hours is preferred and ≤ 3 hours are more preferred.

However, although the added aldehyde is not completely converted during step (a), which means that still unconverted aldehyde is present, the reaction mixture contains besides the desired saturated aliphatic carboxylic acid also byproducts with a high oxidation potential, which have been formed by peroxidation. Such byproducts are for example formed when oxygen as O₂ is wholly inserted into the aldehyde to form a peroxy acid as shown in the following reaction equation 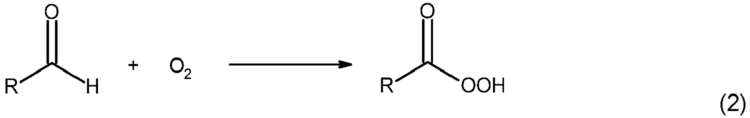 in which R denotes a C₂₋₄ radical. Besides the above-mentioned peroxy acid, also other byproducts with a high oxidation potential like alkyl hydroperoxides may be formed, albeit usually in small amounts compared to the peroxy acid. Since their adverse effects are mainly based on their oxidation potential as such, such by-products are generally characterized by their content of so-called "active oxygen" as a quantitative measure of the amount of reactive oxygen which they would supply to easily oxidable compounds. The term "active oxygen" is already known and used in the state of the art, and is for example described in A. Uhl et al., "Peroxy Compounds, Organic" in Ullmann's Encyclopedia of Industrial Chemistry, 2017, Wiley-VCH Verlag GmbH & Co. KGaA, DOI: 10.1002/14356007.a19_199.pub2, chapter 10 "Analytical Determination". The amount of active oxygen of a sample is generally determined by adding a defined amount of an easily oxidable compound, such as salts of iodide(1-) or iron(II), to a defined amount of the sample, which are then oxidized by the compounds with high oxidation potential.

According to the present invention, the determination of active oxygen is preferably performed by oxidation of iodide(1-). This analytical method is called iodometry and is well known by the person skilled in the art. However, it is also roughly explained below.

At the iodometry, a defined amount of an aqueous solution of potassium iodide in acetic acid is added to a defined amount of the sample at room temperature and stirred in order to oxidize the iodide(1-) to elemental iodine. The amount of added potassium iodide relates to the expected amount of active oxygen and can be estimated by preliminary measures. For the iodometric measurement, the amount of the added iodide(1-) has to be a bit higher than the amount oxidized to elemental iodine. The elemental iodine is then titrated with sodium thiosulfate in order to determine the amount of elemental iodine which was formed by the previous oxidation. As an indicator, starch is typically used, which is violet as long as elemental iodine is present and which gets colorless when all the elemental iodine was reduced to iodide. Alternatively, also a platinum electrode can be used. Based on the amount of added potassium iodide and the amount of elemental iodine formed by the oxidation, the amount of the oxidized iodide(1-) can be calculated. According to the formal equation

2I⁻ + 2H⁺ + "O" → I₂ + H₂O (3)

and the more detailed equation 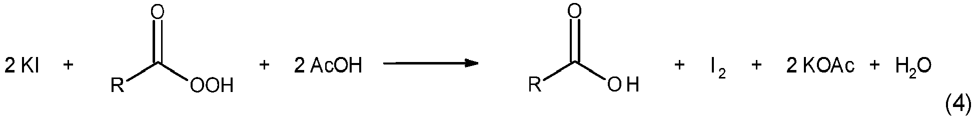 two moles of iodide(1-) react in the presence of acetic acid with one mole of active oxygen atoms. Active oxygen is indicated in formula (3) by "O" and is part of the peroxo group in formula (4). It is reduced to water. In equation (4), R denotes a C₂₋₄ radical, and "Ac" stands for an acetyl group. The active oxygen content of the sample is the weight fraction of the active oxygen atoms based on the weight of the sample and specified in wt.-% or wt.-ppm. The content of active oxygen can easily be converted into an equivalent content of the peroxy acid, assuming that the active oxygen would solely be bound in the peroxy acid, by multiplying the active oxygen content by the ratio of the molar mass of the peroxy acid to the molar mass of an oxygen atom. In case of perpropionic acid, the multiplication factor is 90.0/16.0 = 5.625.

For the sake of completeness, it is also mentioned that the amount of active oxygen in carboxylic acid containing samples can basically also be determined by physical methods such as ¹³C-NMR. Regarding peroxy acids, the carbon signal of the peracid group appears approximately at 170 ppm, whereas the carbon signal of the acid group appears approximately at 180 ppm.

However, within the present invention, active oxygen is understood as the mass of oxygen present in the sample which is capable to oxidize iodide(1-) in aqueous acetic acid medium at room temperature and atmospheric pressure to elemental iodine.

The active oxygen content of the mixture obtained in step (a) is typically 0.02 to 1 wt.-% based on the mixture, preferably ≥ 0.03 wt.-% and more preferably ≥ 0.05 wt.-%, and preferably ≤ 0.8 wt.-% and more preferably ≤ 0.5 wt.-%.

The composition of the reaction mixture of step (a) regarding the content of the saturated aliphatic carboxylic acid and the corresponding aldehyde can usually be determined by gas chromatography.

After a mixture containing the saturated aliphatic carboxylic acid and ≤ 2 mol-% of the corresponding aldehyde with respect to the saturated aliphatic carboxylic acid is obtained in step (a), the reaction liquid and the remaining oxygen containing gas are preferably separated. This may for example be done in a batch process by discharging the oxygen containing gas phase, or in a continuous process by removing the liquid reaction mixture from the reaction device.

It was surprisingly found that the active oxygen content of the mixture obtained in step (a) can be significantly reduced if the mixture is thermally treated in a subsequent step, which is named step (b), in the liquid phase at a temperature of 80 to 250°C and a pressure of 0.1 to 2 MPa abs for 0.25 to 100 hours. To avoid the formation of additional active oxygen containing compounds, the thermal treatment is particularly preferably performed without addition of further oxygen.

Basically, the typical reactor for step (b) is a residence time container in which the liquid mixture is kept at a defined temperature range and a defined pressure range for a defined time. Depending on whether the process for the preparation of the saturated aliphatic carboxylic acid is performed discontinuously or semi-continuously at the one hand, or continuously at the other hand, different types of reactors are advantageous.

For a discontinuous or semi-continuous preparation process, the mixture obtained in step (a) can, for example, be kept in the reactor in which the conversion of the aldehyde took place and the conditions for the thermal treatment according to step (b) be adjusted. As already mentioned above, typical reactors for the discontinuous or semi-continuous oxidation of the aldehyde are mainly autoclaves. However, it is, of course, also possible to transfer the reaction mixture obtained in step (a) into a separate residence time container such as a stirred or not stirred tank.

For a continuous preparation process, the reaction mixture obtained in step (a) is typically continuously fed through the residence time container, which usually is a separate container. Without limiting the scope, the residence time container can, for example, be a tubular reactor or a cascade of stirred tank reactors. Their geometry is selected in such a way that the desired residence time is achieved while the reaction mixture continuously flows through it. In terms of the present invention regarding step (b), the term tubular reactor does also embrace elongated tubes whose length is significantly longer than their diameter and which are commonly also described as pipes due to their high length/diameter ratio. Although the length/diameter ratio is not an essential parameter for the present invention, the tubular reactors preferably have a length/diameter ratio of 1 to 25, more preferably of ≥ 2 as well as more preferably of ≤ 15. Back-mixing shall be advantageously avoided or at least suppressed since otherwise the effect of the thermal treatment would be contradicted or reduced. One means for avoiding or suppressing back-mixing is, for example, choosing the geometry of the tubular reactor in a way that more or less a plug flow results. Even if the geometry of the tubular reactor would not be sufficient to facilitate an adequate plug flow, a suitable plug flow can still be achieved by providing internals, such as perforated plates or random packings like rings, Raschig rings, Pall rings, Ralu rings, torus saddles and the such. If a cascade of stirred tank reactors is used, it is recommended to use a cascade with at least three stirred tank reactors to reduce the back-mixing. Since the complexity of the residence time containers increase with an increasing number of stirred tanks, it is preferred to use less than ten, more preferred to use less than eight, and particularly preferred to use less than five stirred tanks.

In a preferred embodiment of the present invention, the preparation of the saturated aliphatic carboxylic acid is performed continuously, and the thermal treatment in step (b) is performed in a tubular reactor. More preferably, such tubular reactor is substantially free of internals.

The thermal treatment may be carried out in the presence or absence of a decomposition catalyst. If a decomposition catalyst is used, it will normally be a homogeneous catalyst, whereby heterogeneous catalysts are, however, also not excluded. As examples of homogeneous decomposition catalysts, soluble salts of transition metals of groups 6 to 11, preferably of groups 6 to 9, and more preferably of the first row of groups 6 to 9 of the Periodic Table of the Elements are mentioned. Particularly preferably, the homogeneous decomposition catalysts contain Mn, Fe or Co, and very particularly preferably Mn. The concentration of the homogeneous decomposition catalyst metals may vary in a broad range, but a metal content of 0,0001 to 0,1 wt.-% based on the mixture to be treated is a typical content. Although decomposition catalysts enhance the decomposition of the active oxygen containing compounds, they generally have the disadvantage that a further component has to be added to the mixture which is to be treated, and afterwards be separated off to obtain the saturated aliphatic carboxylic acid in step (c) free of the homogeneous catalyst. It was found within the present invention that at the specified conditions of a temperature of 80 to 250°C, a pressure of 0.1 to 2 MPa abs, and a decomposition time of 0.25 to 100 hours, the decomposition sufficiently and consistently also takes place without the addition of a homogeneous catalyst.

The thermal treatment for decomposing the active oxygen containing compounds is performed at a temperature of 80 to 250°C and a pressure of 0.1 to 2 MPa abs. It is preferably carried out at a temperature of ≥ 90°C, more preferably at ≥ 100°C, and preferably at ≤ 200°C, more preferably at ≤ 180°C. With regard to the pressure, it is generally preferred to perform the thermal treatment at a pressure which is at least equal to, or more preferably higher than the vapor pressure of the saturated aliphatic carboxylic acid at the treatment temperature in order to keep the saturated aliphatic carboxylic acid almost completely in the liquid phase. Particularly preferably the pressure is ≥ 1.2 times the vapor pressure of the saturated aliphatic carboxylic acid at the treatment temperature. Particularly, the thermal treatment in step (b) is performed at a temperature of 100 to 180°C.

Since the initial concentration of the active oxygen containing compounds is rather low, the adiabatic temperature increase is usually less than 10 K and thus it is generally not necessary to remove the reaction heat. Therefore, the thermal treatment is usually performed adiabatically or quasi-adiabatically. It also can be performed isothermally, or the temperature altered, e.g. raised, during the thermal treatment.

As already mentioned above, the thermal treatment is particularly preferably performed without addition of further oxygen. If the process is carried out continuously and the thermal treatment performed in a tubular reactor, no gaseous phase is normally present since the liquid reaction mixture obtained in step (a) fully fills the space of the tubular reactor. If the thermal treatment is performed in a way that there is a gas phase present, which might be the case for a thermal treatment in stirred vessels, it is advantageous to apply a gaseous phase free or substantially free of oxygen. Preferably, the gaseous phase mostly contains inert gases such as nitrogen, noble gases or mixtures thereof.

Depending on the nature of the active oxygen containing compounds, their amount, the treatment conditions such as the treatment temperature, and the active oxygen content which is aimed to be achieved, the residence time under the thermal treatment conditions is in the range of 0.25 to 100 hours. In general, a low active oxygen content of the mixture obtained in step (a), a high temperature during the thermal treatment of up to 250°C and a higher tolerance for the aimed active oxygen content at the end of the thermal treatment, lead to a shorter residence time, whereas a high active oxygen content of the mixture obtained in step (a), a low temperature during the thermal treatment and a lower tolerance for the aimed active oxygen content at the end of the thermal treatment, lead to a longer residence time. It was recognized according to the invention that the treatment temperature commonly has a great influence on the rate of the decomposition of the active oxygen containing compounds. In the course of the invention, it was, for instance, found by tests with a propionic acid containing system that it took 6 hours at 100°C to decompose 90% of the active oxygen containing compounds, whereas the same degree of decomposition was already achieved after 0.5 hours at 130°C.

The thermal treatment in step (b) is preferably performed for ≥ 0.1 hours, more preferably for ≥ 0.25 hours, and preferably for ≤ 100 hours, more preferably for ≤ 10 hours and particularly preferably for ≤ 5 hours. Particularly, the thermal treatment in step (b) is performed for 0.25 to 5 hours.

The active oxygen content of the thermally treated mixture obtained in step (b) is typically 0 to 100 wt.-ppm based on the thermally treated mixture, preferably ≤ 75 wt.-ppm and more preferably ≤ 50 wt.-ppm. It is expressly emphasized that the active oxygen content of the thermally treated mixture obtained in step (b) can already be very low, depending on the active oxygen content of the mixture obtained in step (a) and the conditions under which the thermal treatment in step (b) was performed.

The thermally treated mixture obtained in step (b) is then distilled in step (c) in a distillation apparatus to obtain a distillate containing ≥ 90 wt.-% of the saturated aliphatic carboxylic acid and having an active oxygen content of 0 to 25 wt.-ppm based on the distillate.

The distillation apparatus is a single distillation column. Usually, low boiling components and high boiling components are separated off from the desired saturated aliphatic carboxylic acid. This can either be done in one distillation column in which the low boilers are separated off overhead, the high boilers separated off as bottom product and the saturated aliphatic carboxylic acid is withdrawn as a side stream.

Irrespective of whether only one distillation column or an interconnection of two or more distillation columns is used, distillation columns known in the state of the art can be used. They usually have a bottom evaporator, a top condenser and internals. The column body can, for example, be equipped with structured packings, random packings or trays. The number of separation stages required is mainly dependent on the separation task, and particularly on the differences of the boiling points of the saturated aliphatic carboxylic acid in relation to those of the low boilers and the high boilers, as well as on the aimed purity of the saturated aliphatic carboxylic acid. The distillation columns can easily be designed with the skills of the person skilled in the art.

If two or more saturated aliphatic carboxylic acids are prepared together in the preparation process, the number of the required distillation columns is normally equal to, or one more than the number of different saturated aliphatic carboxylic acids to be purified.

The distillative separation of the saturated aliphatic carboxylic acid can be performed continuously as well as discontinuously. In case of a continuous oxidation process in step (a), it is usually advantageous to also continuously perform step (b) and (c). Conversely, it is usually advantageous to perform step (b) and (c) discontinuously if the oxidation process in step (a) is also performed discontinuously.

In addition to the design of the distillation columns, also their operation conditions can be easily determined with the skills of the person skilled in the art. For practical reasons, the distillation columns are advantageously designed and operated in a way that the evaporation of the mixture fed into the respective distillation column can be effected by use of what is called "16 bar steam" (1.6 MPa steam), which is normally available at chemical plant sites, and that the condensation of the overhead products can be effected with cooling water. This leads to a preferred evaporation temperature of < 180°C and a preferred condensation temperature of > 40°C. Furthermore, it is advantageous within the above-mentioned data to design and operate the distillation columns in a way that no or only a slight heating or cooling of the feed stream is required. Regarding the distillation column, to which the thermally treated mixture obtained in step (b) is fed to, it is preferably designed in a way that besides the use of 16 bar steam and cooling water, it can be directly operated at or almost at the same pressure for which the temperature in the tower at the feeding point is close to the temperature of the stream exiting the thermal treatment, preferably within ± 10 K and more preferably within ± 5°K. Furthermore, it is also advantageous to operate the distillation column, to which the thermally treated mixture obtained in step (b) is fed to, at the same or almost the same temperature under which the thermally treated mixture is obtained. It is specifically mentioned that the above-mentioned adaption of the temperature and pressure of the distillation column to that of the thermally treated mixture can often easily be achieved by already choosing the temperature and pressure of the thermal treatment in step (b) in a way that the thermally treated mixture can easily be fed to the distillation in step (c) without significantly adapting the temperature and pressure.

The above-mentioned process enables the preparation of the saturated aliphatic carboxylic acid in a high purity with a content of ≥ 90 wt.-% of the saturated aliphatic carboxylic acid and of 0 to 25 wt.-ppm of active oxygen based on the distillate. The content of the saturated aliphatic carboxylic acid is preferably ≥ 95 wt.-%, more preferably ≥ 98 wt.-%, particularly preferably ≥ 99 wt.-%, and very particularly preferably ≥ 99.5 wt.-% based on the distillate.

As typical byproducts, formates, carboxylic acids with a lower number of carbon atoms, alcohols, ketones and water are mentioned.

The active oxygen content of the distilled saturated aliphatic carboxylic acid obtained in step (c) is preferably ≤ 20 wt.-ppm, more preferably ≤ 15 wt.-ppm, particularly preferably ≤ 10 wt.-ppm and very particularly preferably ≤ 8 wt.-ppm. Although an active oxygen content of 0 wt.-% can be achieved, it is frequently ≥ 1 wt.-ppm.

In order to preserve the very low content of active oxygen, as well as the very low content of oxygen dissolved in the saturated aliphatic carboxylic acid, it is advantageous to store, handle and ship the purified saturated aliphatic carboxylic acid under a blanket of inert gas, such as nitrogen, noble gases or mixtures thereof.

As already mentioned before, each step of steps (a) to (c) can be performed continuously or discontinuously, whereas for step (a) also a semi-continuous operation is an additional option. It may be advantageous for lower production volumes to produce the saturated aliphatic carboxylic acid in a discontinuous or semi-continuous process since such discontinuous and semi-continuous processes are normally more flexible and easier to operate for small production volumes. On the other hand, a continuous process in which steps (a) to (c) are performed continuously has the advantage of being more efficient as soon as the process is started up and stably running. It is therefore a preferred option to prepare the saturated aliphatic carboxylic acid continuously. This particularly applies for saturated aliphatic carboxylic acids which are produced in large volumes, as for example for propionic acid, n-butyric acid and n-pentanoic acid.

In a general embodiment for the continuous preparation of propionic acid, liquid propionaldehyde and gaseous oxygen are continuously fed into a jet loop reactor, which is operated at a temperature in the range of 60 to 80°C and at an oxygen partial pressure in the range of 0.1 to 0.5 MPa. To remove the heat produced by the exothermal oxidation reaction, the reactor is externally cooled by an external heat exchanger. The reaction mixture obtained by the above-mentioned step (a) still contains non-converted propionaldehyde in an amount of 0.1 to 2 mol-% with respect to propionic acid, and active oxygen in an amount of 0.02 to 1 wt.-% based on the mixture. It continuously passes a heat exchanger, in which it is heated to a temperature in the range of 100 to 180°C, and fed into a compartmented tubular reactor, through which the reaction mixture flows within 0,25 to 2 hours in a plug flow. The tubular reactor is operated at a total pressure of 0,1 to 1 MPa abs. The thermally treated mixture obtained by the above-mentioned step (b) has a significantly reduced active oxygen content of only 1 to 100 wt.-ppm. The thermally treated mixture is then continuously fed into a distillation column, in which high boilers and low boilers like residual propionaldehyde and acetic acid are separated off, and purified propionic acid withdrawn as a side stream. The propionic acid obtained by the above-mentioned step (c) has a high purity and contains ≥ 99 wt.-% propionic acid and only 1 to 25 wt.-ppm active oxygen based on the distillate.

In a general embodiment for the discontinuous preparation of 2-methylpropionic acid (isobutyric acid), a tempered (heatable and coolable) autoclave is filled with a mixture of liquid 2-methylpropionaldehyde (isobutyric aldehyde) and 2-methylpropionic acid in equal amounts, and 4 wt.-% of potassium isobutyrate are added. The reactor is closed, heated to a temperature in the range of 60 to 80°C and pressurized with nitrogen to a pressure of 1 to 2.5 MPa abs with stirring. The reaction is started by adding oxygen in a pressure-controlled manner and in such a way that the additional oxygen pressure is at most 10% of the initial nitrogen pressure. The reaction is continued until nearly no more oxygen is uptaken and the total pressure remains essentially constant. The reactor is then depressurized and flushed with nitrogen until the off-gas contains less than 10 vol.-ppm of oxygen and repressurized with nitrogen. The reaction mixture obtained by the above-mentioned step (a) still contains non-converted 2-methylpropionaldehyde in an amount of 0.1 to 2 mol-% with respect to 2-methylpropionic acid, and active oxygen in an amount of 0.02 to 1 wt.-% based on the mixture. It is then thermally treated in the autoclave at a temperature in the range of 100 to 150°C and a total pressure of 0,1 to 1 MPa abs for 0,25 to 2 hours. The autoclave is then depressurized. The thermally treated mixture obtained by the above-mentioned step (b) has a significantly reduced active oxygen content of only 1 to 100 wt.-ppm. The thermally treated mixture is then transferred into a batch distillation apparatus and distilled. The fraction of the purified 2-methylpropionic acid has a high purity and contains ≥ 99 wt.-% 2-methylpropionic acid and only 1 to 25 wt.-ppm active oxygen based on the distillate.

The process of the invention enables the preparation of saturated aliphatic carboxylic acids with 3 to 5 carbon atoms by oxidation of the corresponding aldehyde with oxygen in high yield and high purity, and particularly without or at least with a very low content of peroxy acid and other peroxides. The process can also be easy operated and functions stably over long operating times and producing the saturated aliphatic carboxylic acids in a constant high quality.

### Examples

### Iodometry

Approximately 5 g of the sample, weighed to the nearest 0.1 mg, are placed in a normed reaction vial, flushed with argon and dissolved in 40 ml of a 1:1 acetic acid / chloroform mixture. The reaction vial is provided with a cooler and placed in a stirring heating block, that is already preheated to 80°C. A weak argon flow is passed through the cooler to cover the sample surface. This is necessary to avoid the ingress of atmospheric oxygen. Afterwards, 5.0 mL of a saturated potassium iodide solution (ca. 60.0 g potassium iodide dissolved in 100 mL deionized water) are added through the cooler and the mixture is boiled under reflux for 10 min. In the next step, 40.0 mL deionized water are added, and the sample solution is titrated with a 0.01 M thiosulfate solution while using a platinum electrode.

### Example 1

### (Preparation of crude propionic acid)

Crude propionic acid was produced in a technical plant with a production capacity of around 4 tons propionaldehyde per hour by oxidation of propionaldehyde with air. The technical plant comprised three reactors which were connected in series. The first two reactors were fully back-mixed and nearly isothermal jet-loop reactors and the third reactor was an adiabatic sieve plate tower with a nearly ideal tube characteristic. The oxidation was performed with a slight excess of air, providing 105 to 110% of the theoretical amount of O₂ based on propionaldehyde, or in other words, providing a molar ration of oxygen to propionaldehyde of 0.525 to 0.55. Propionaldehyde was only fed to the first reactor and air only to the first two reactors with a distribution of approximately 90% of the total amount for the first reactor and the remaining 10% for the second reactor. All three reactors were operated at a temperature of 75°C and a pressure of 2.2 MPa abs. The propionaldehyde conversion at the outlet of the third reactor was > 99% based on the amount of propionaldehyde fed into the first reactor.

The crude propionic acid obtained at the exit of the third reactor contained 98.4 wt.-% propionic acid and 650 wt.-ppm of active oxygen, measured by iodometry as described above. It was filled, stored and shipped under an inert gas atmosphere.

### Example 2

### (Comparative example)

A sample of 1 kg of the crude propionic acid of example 1 was distilled in a batch distillation apparatus containing a 2 m column packed with mesh rings. The column was operated at a top pressure of 0.123 MPa abs and propionic acid distilled off overhead and the fraction boiling at 147.5 ± 0.5°C was collected. The recovered propionic acid fraction had a propionic acid content of 99.8 wt.-%, analyzed by gas chromatography, and contained 26 wt.-ppm active oxygen, measured by iodometry as described above.

The propionic acid fraction obtained by the above-mentioned batch distillation showed the same propionic acid content and the same active oxygen content as the propionic acid fraction obtained in the technical plant by distillation in the commercial distillation tower of technical size.

### Example 3

### (Example according to the invention)

Another sample of 1 kg of the crude propionic acid of example 1 was heated in a glass flask under a nitrogen atmosphere to 105°C and kept under these conditions for 6 hours. By this procedure, the active oxygen content decreased from 650 wt.-ppm of the crude propionic acid to 89 wt.-ppm at the end of the thermal treatment. The thermally treated mixture was then distilled in the same batch distillation apparatus and under the same conditions as described in example 2. The recovered propionic acid fraction had a propionic acid content of 99.8 wt.-%, analyzed by gas chromatography, and contained 4 wt.-ppm active oxygen, measured by iodometry as described above.

### Example 4

### (Example according to the invention)

A further sample of 1 kg of the crude propionic acid of example 1 was admixed with manganese propionate to achieve a Mn concentration in the mixture of 0.34 wt.-ppm. The mixture was then heated in a glass flask under a nitrogen atmosphere to 100°C and kept under these conditions for 6 hours. By this procedure, the active oxygen content decreased from 650 wt.-ppm of the crude propionic acid to 22 wt.-ppm at the end of the thermal treatment. The thermally treated mixture was then distilled in the same batch distillation apparatus and under the same conditions as described in example 2. The recovered propionic acid fraction had a propionic acid content of 99.8 wt.-%, analyzed by gas chromatography, and contained 3 wt.-ppm active oxygen, measured by iodometry as described above.

Comparative example 2 shows that without the inventive thermal treatment process, the active oxygen content of the distilled propionic acid amounts to significant 26 wt.-ppm, whereas the inventive thermal treatment process performed before the final distillation enables an active oxygen content of the distilled propionic acid of very low 4 wt.-ppm in inventive example 3 and very low 3 wt.-ppm in inventive example 4.

The examples also show that a homogeneously catalyzed decomposition by manganese propionate leads to nearly the same result as the non-catalyzed decomposition. Thus, it is shown that the inventive process does already perform in the absence of a catalyst.

## Claims

1. A process for the preparation of a saturated aliphatic non-alpha-branched carboxylic acid with 3 to 5 carbon atoms by oxidation of the corresponding aldehyde with oxygen, which comprises
(a) converting the corresponding aldehyde with oxygen at a temperature of 40 to 150°C and an oxygen partial pressure of 0.001 to 1 MPa to obtain a mixture containing the saturated aliphatic carboxylic acid and ≤ 2 mol-% of the corresponding aldehyde with respect to the saturated aliphatic carboxylic acid,
(b) thermally treating the mixture obtained in step (a) in the liquid phase at a temperature of 80 to 250°C and a pressure of 0.1 to 2 MPa abs for 0.25 to 100 hours, and
(c) distilling the mixture obtained in step (b) in a single distillation column to obtain a distillate containing ≥ 90 wt.-% of the saturated aliphatic carboxylic acid and having an active oxygen content of 0 to 25 wt.-ppm based on the distillate.

2. The process according to claim 1, wherein the saturated aliphatic non-alpha-branched carboxylic acid is prepared by converting in step (a) propionaldehyde, n-butyraldehyde or n-pentanaldehyde and conducting the oxidation in the absence of salts of alkali metals, of alkaline earth metals and of transition metals of group 12 of the Periodic Table of the Elements.

3. The process according to any of claims 1 to 2, wherein the saturated aliphatic non-alpha-branched carboxylic acid is propionic acid and the aldehyde is propionaldehyde.

4. The process according to any of claims 1 to 3, wherein the active oxygen content of the mixture obtained in step (a) is 0.02 to1 wt.-% based on the mixture.

5. The process according to any of claims 1 to 4, wherein the preparation of the saturated aliphatic non-alpha-branched carboxylic acid is performed continuously, and the thermal treatment in step (b) is performed in a tubular reactor.

6. The process according to any of claims 1 to 5, wherein the thermal treatment in step (b) is performed at a temperature of 100 to 180°C.

7. The process according to any of claims 1 to 6, wherein the thermal treatment in step (b) is performed for 0.25 to 5 hours.

8. The process according to any of claims 1 to 7, wherein the active oxygen content of the thermally treated mixture obtained in step (b) is 0 to 100 wt.-% based on the thermally treated mixture.

9. The process according to any of claims 1 to 8, wherein a distillate containing ≥ 99 wt.-% of the saturated aliphatic non-alpha-branched carboxylic acid based on the distillate is obtained.

10. The process according to any of claims 1 to 9, wherein a distillate containing the saturated aliphatic non-alpha-branched carboxylic acid has an active oxygen content of 0 to 10 wt.-ppm based on the distillate.

11. The process according to any of claims 1 to 10, wherein the saturated aliphatic non-alpha-branched carboxylic acid is prepared continuously.

## Patentansprüche

1. Verfahren zur Herstellung einer gesättigten aliphatischen nicht alpha-verzweigten Carbonsäure mit 3 bis 5 Kohlenstoffatomen durch Oxidation des entsprechenden Aldehyds mit Sauerstoff, das Folgendes umfasst:
(a) Umwandeln des entsprechenden Aldehyds mit Sauerstoff bei einer Temperatur von 40 bis 150 °C und einem Sauerstoff-Partialdruck von 0,001 bis 1 MPa unter Erhalt eines Gemischs, das die gesättigte aliphatische Carbonsäure und ≤ 2 Mol-% des entsprechenden Alkylens, bezogen auf die gesättigte aliphatische Carbonsäure, enthält,
(b) Wärmebehandeln der in Schritt (a) erhaltenen Mischung in der Flüssigphase bei einer Temperatur von 80 bis 250 °C und einem Druck von 0,1 bis 2 MPa abs. über einen Zeitraum von 0,25 bis 100 Stunden und
(c) Destillieren der in Schritt (b) erhaltenen Mischung in einer einzigen Destillationskolonne unter Erhalt eines Destillats, das ≥ 90 Gew.-% der gesättigten aliphatischen Carbonsäure enthält und einen Gehalt an aktivem Sauerstoff von 0 bis 25 Gew.-ppm, bezogen auf das Destillat, aufweist.

2. Verfahren nach Anspruch 1, bei dem die gesättigte aliphatische nicht alpha-verzweigte Carbonsäure hergestellt wird, indem in Schritt (a) Propionaldehyd, n-Butyraldehyd oder n-Pentanaldehyd umgesetzt und die Oxidation in Abwesenheit von Salzen von Alkalimetallen, Erdalkalimetallen und Übergangsmetallen der Gruppe 12 des Periodensystems der Elemente durchgeführt wird.

3. Verfahren nach einem der Ansprüche 1 bis 2, bei dem es sich bei der gesättigten aliphatischen nicht alpha-verzweigten Carbonsäure um Propionsäure handelt und es sich bei dem Aldehyd um Propionaldehyd handelt.

4. Verfahren nach einem der Ansprüche 1 bis 3, bei dem der Gehalt des in Schritt (a) erhaltenen Gemischs an aktivem Sauerstoff 0,02 bis 1 Gew.-%, bezogen auf das Gemisch, beträgt.

5. Verfahren nach einem der Ansprüche 1 bis 4, bei dem die Herstellung der gesättigten aliphatischen nicht alpha-verzweigten Carbonsäure kontinuierlich durchgeführt wird und die Wärmebehandlung in Schritt (b) in einem Rohrreaktor durchgeführt wird.

6. Verfahren nach einem der Ansprüche 1 bis 5, bei dem die Wärmebehandlung in Schritt (b) bei einer Temperatur von 100 bis 180 °C durchgeführt wird.

7. Verfahren nach einem der Ansprüche 1 bis 6, bei dem die Wärmebehandlung in Schritt (b) in einen Zeitraum von 0,25 bis 5 Stunden durchgeführt wird.

8. Verfahren nach einem der Ansprüche 1 bis 7, bei dem der Gehalt des in Schritt (b) erhaltenen wärmebehandelten Gemischs an aktivem Sauerstoff 0 bis 100 Gew.-%, bezogen auf das wärmebehandelte Gemisch, beträgt.

9. Verfahren nach einem der Ansprüche 1 bis 8, bei dem ein Destillat erhalten wird, das ≥ 99 Gew.-% der gesättigten aliphatischen nicht alpha-verzweigten Carbonsäure, bezogen auf das Destillat, enthält.

10. Verfahren nach einem der Ansprüche 1 bis 9, bei dem ein Destillat, das die gesättigte aliphatische nicht alpha-verzweigte Carbonsäure enthält, einen Gehalt an aktivem Sauerstoff von 0 bis 10 Gew.-ppm, bezogen auf das Destillat, aufweist.

11. Verfahren nach einem der Ansprüche 1 bis 10, bei dem die gesättigte aliphatische nicht alpha-verzweigte Carbonsäure kontinuierlich hergestellt wird.

## Revendications

1. Procédé pour la préparation d'un acide carboxylique aliphatique saturé non ramifié en alpha comportant 3 à 5 atomes de carbone par oxydation de l'aldéhyde correspondant avec de l'oxygène, qui comprend
(a) la conversion de l'aldéhyde correspondant avec de l'oxygène à une température de 40 à 150 °C et une pression partielle d'oxygène de 0,001 à 1 MPa pour obtenir un mélange contenant l'acide carboxylique aliphatique saturé et ≤ 2 % en moles de l'aldéhyde correspondant par rapport à l'acide carboxylique aliphatique saturé,
(b) le traitement thermique du mélange obtenu dans l'étape (a) dans la phase liquide à une température de 80 à 250 °C et une pression de 0,1 à 2 MPa absolu pendant 0,25 à 100 heures, et
(c) la distillation du mélange obtenu dans l'étape (b) dans une unique colonne de distillation pour obtenir un distillat contenant ≥ 90 % en poids de l'acide carboxylique aliphatique saturé et ayant une quantité d'oxygène actif de 0 à 25 ppm en poids sur la base du distillat.

2. Procédé selon la revendication 1, l'acide carboxylique aliphatique saturé non ramifié en alpha étant préparé par conversion dans l'étape (a) de propionaldéhyde, de n-butyraldéhyde ou de n-pentanaldéhyde et conduite de l'oxydation en l'absence de sels de métaux alcalins, de métaux alcalino-terreux et de métaux de transition du groupe 12 du tableau périodique des éléments.

3. Procédé selon l'une quelconque des revendications 1 à 2, l'acide carboxylique aliphatique saturé non ramifié en alpha étant l'acide propionique et l'aldéhyde étant le propionaldéhyde.

4. Procédé selon l'une quelconque des revendications 1 à 3, la teneur d'oxygène actif du mélange obtenu dans l'étape (a) étant de 0,02 à 1 % en poids sur la base du mélange.

5. Procédé selon l'une quelconque des revendications 1 à 4, la préparation de l'acide carboxylique aliphatique saturé non ramifié en alpha étant réalisée de manière continue, et le traitement thermique dans l'étape (b) étant réalisé dans un réacteur tubulaire.

6. Procédé selon l'une quelconque des revendications 1 à 5, le traitement thermique dans l'étape (b) étant réalisé à une température de 100 à 180 °C.

7. Procédé selon l'une quelconque des revendications 1 à 6, le traitement thermique dans l'étape (b) étant réalisé pendant 0,25 à 5 heures.

8. Procédé selon l'une quelconque des revendications 1 à 7, la teneur d'oxygène actif du mélange traité thermiquement obtenu dans l'étape (b) étant de 0 à 100 % en poids sur la base du mélange traité thermiquement.

9. Procédé selon l'une quelconque des revendications 1 à 8, un distillat étant obtenu, contenant ≥ 99 % en poids de l'acide carboxylique aliphatique saturé non ramifié en alpha sur la base du distillat.

10. Procédé selon l'une quelconque des revendications 1 à 9, un distillat contenant l'acide carboxylique aliphatique saturé non ramifié en alpha ayant une teneur d'oxygène actif de 0 à 10 ppm en poids sur la base du distillat.

11. Procédé selon l'une quelconque des revendications 1 à 10, l'acide carboxylique aliphatique saturé non ramifié en alpha étant préparé de manière continue.
